# EUROPÄISCHE PATENTANMELDUNG

(11) **EP 0 762 122 A1**
(43) Veröffentlichungstag der Anmeldung: **12.03.1997**
(21) Anmeldenummer: 96112608.3
(22) Anmeldetag: 05.08.1996
(51) Int. Cl.: G01N 33/543, G01N 33/58

(54) **Optischer Festphasenbiosensor auf Basis von Streptavidin und Biotin**

(30) Priorität: 16.08.1995 DE 19530078
(71) Anmelder: BAYER AG, 51368 Leverkusen (DE)
(72) Erfinder: Diederich, Anke, 67227 Frauenthal (DE); Lösche, Matthias, Prof. Dr., 04229 Leipzig (DE); Völker, Michael, Dr., 51061 Köln (DE); Siegmund, Hans-Ulrich, Dr., 51149 Köln (DE); Heiliger, Ludger, Dr., 51373 Leverkusen (DE)

(57) **Zusammenfassung**

Ein neuer optischer Festphasenbiosensor basierend auf den Förster-Energietransfer zwichen einem Fluoreszenzfarbstoff F1 und F2, beispielsweise in Form eines Teststreifens, besteht aus
a) einem transparenten Träger,
b) einer daraufliegenden Multischicht aus Polyanionen und Polykationen, die als oberste Schicht biotinyliertes Polylysinhydrobromid enthält,
c) einer Abdeckung der obersten ionischen Schicht durch Streptavidin, welches an die biotinylierte Schicht gebunden wird und
d) weiteren biotinylierten Biomolekülen als Rezeptoren,
wobei der Fluoreszenzfarbstoff F1 an das Streptavidin oder an weitere Biomoleküle gebunden sein kann.

Der Analyt ist mit dem Farbstoff F2 ausgerüstet oder kompetitiert mit einem mit F2 ausgerüsteteten und an den Biosensor gebundenen Analyten.

## Beschreibung

Die vorliegende Erfindung betrifft einen optischen Biosensor zur Detektion von gelösten Molekülen (im folgenden Analyten genannt), die sich mit einem Fluoreszenzfarbstoff markieren lassen und für die ein diese spezifisch erkennendes Biomolekül (im folgenden Rezeptor genannt) existiert. Es handelt sich hierbei um einen Festphasensensor mit Fluoreszenzfarbstoff, der über einen Energietransferprozeß auf ein zu detektierendes, mit einem zweiten Fluoreszenzfarbstoff markiertes Molekül die Bestimmung von dessen Anwesenheit und Menge erlaubt. Über eine Verdrängungs- oder eine Sandwich-Reaktion ist die Bestimmung auch von unmarkierten Analyten möglich.

Es gibt verschiedene Methoden, Analyten, wie Hormone, Enzyme, andere Proteine, Kohlenhydrate, Nukleinsäuren, pharmakologische Wirkstoffe, Toxine und andere, in flüssigen Proben biologischen Ursprungs nachzuweisen. Unter den bekannten Methoden ragen speziell Immunoassays und damit verwandte Verfahren als eine empfindliche Nachweismethode zur Bestimmung sehr kleiner Mengen organischer Substanzen heraus. Immunoassay-Methoden beruhen allgemein auf der Fähigkeit eines Rezeptormoleküls, beispielsweise eines Antikörpers, die Struktur und molekulare Organisation eines Ligandenmoleküls, sei sie durch unpolare und/oder polare Wechselwirkungen definiert, spezifisch zu erkennen und dieses Molekül auf derartige Weise ganz spezifisch zu binden.

Immunoassays werden mit verschiedenen Methoden durchgeführt. Dazu zählen der Einsatz verschiedener Markierungstechniken, die oft auf eine Quantifizierung des Analyten mittels radioaktiver, enzymgekoppelter und fluoreszierender Marker abzielen (E. F. Ulman, P. L. Khanna, Methods in Enzymology, 74 (1981) 28-60). Als Sonderfall der letztgenannten Methode kann der strahlungslose Fluoreszenz-Energietransfer (Förster-Energietransfer oder Resonanter Energietransfer, RET) betrachtet werden, mit dem die relative geometrische Position zweier Fluoreszenzfarbstoffe vermessen werden kann, sofern der gegenseitige Abstand höchstens wenige nm beträgt. Damit kann daher die unmittelbare Wechselwirkung eines Rezeptor/Ligand-Paares direkt nachgewiesen werden (L. Stryer, Annual Reviews in Biochemistry 47 (1978) 819-846). In der Technik der Immunoassays und der Biosensorik hat dieses Prinzip mehrfach Erwähnung gefunden (S. M. Barnard, D. R. Walt, Science 251, 927 (1991), EP 150 905, DE 3938598).

Darüber hinaus betrifft die vorliegende Erfindung die Immobilisierung von Biomolekülen in molekular dünnen, wohlgeordneten Schichtstrukturen, die für einen optischen Biosensor mit dem Förster-Energietransfer als Detektionsprinzip besonders geeignet sind. Die Immobilisierung von Rezeptoren an eine Festphase ist für Biosensoren von entscheidender Bedeutung. Nach derzeitiger Technik werden Proteine meist adsorptiv über ionische oder hydrophobe Wechselwirkungen an Oberflächen gebunden oder sie werden über kovalente Bindungen unter Verwendung von Hilfsreagenzien angekoppelt. Als ein mittlerweile klassisch zu nennendes Beispiel für letztere Vorgehensweise sei die Aktivierung von Glas durch 3-Aminopropyl-triethoxysilan und die anschließende Bindung von Protein mit Glutardialdehyd unter Reduktion der entstehenden Schiff-Base durch Natriumborhydrid zu nennen. Einen Überblick über bei Immunoassays benutzte Verfahren findet man beispielsweise in P. Tijssen, "Practice and Theory of Enzyme Immunoassays", S. 297-328 (Elsevier, Amsterdam 1987). Für biotechnologische Verfahren sind daneben Einkapselungsverfahren für Enzyme in permeablen Polymeren oder Membranen gebräuchlich.

Während die adsorptiv arbeitenden Verfahren den Nachteil der mangelnden Stabilität der Proteinimmobilisierung besitzen, erfordert die kovalente Anbindung mit Kopplungs- oder Aktivierungsreagenzien oft eine relativ hohe Anzahl von Prozeßschritten, die Verwendung von hochreinen, teilweise instabilen Reagenzien oder die Anwendung von Umsetzungsbedingungen, unter denen nicht alle Proteine stabil sind. Die meisten gängigen Immobilisierungstechniken haben das Problem gemeinsam, daß die Rezeptoren nicht regiospezifisch gebunden werden, sodaß die für Folgereaktionen wichtigen Molekülteile bei einem hohen Prozentsatz der Rezeptoren sterisch blockiert sind. Auch ist der Wirkungsgrad der Proteinimmobilisierung oft mangelhaft, entweder durch Proteindenaturierung oder durch eine zu geringe Belegung der Oberfläche mit Proteinen. Weiterhin sind einige Aktivierungsreagenzien zur Quervernetzung fähig, wodurch sich schlecht definierte Oberflächen ergeben. Die Reproduzierbarkeit der Immobilisierung wird damit ebenfalls sehr schlecht. Für die Quantifizierung der Analyten-Konzentration mittels Förster-Energietransfer wirkt sich dabei ungünstig aus, daß der Abstand zwischen Energie-Donor und -Akzeptor je nach lokaler Oberflächenbeschaffenheit irregulär variiert, was generell eine Erhöhung der systembedingten Meßungenauigkeit mit sich bringt.

Eine Möglichkeit, die oben genannten systembedingten Probleme zu lösen ist es, den Träger mit molekular wohldefinierten Filmen zu beschichten. Dies kann dadurch geschehen, daß der Träger mit einem dünnen filmbildenden Copolymer überzogen wird, welches neben strukturbildenden Einheiten Comonomere mit Reaktivgruppen in der Seitenkette enthält, welche zur kovalenten Bindung des zu immobilisierenden Proteins in der Lage sind, wie z.B. in DE 43 19 037 beschrieben. Nachteilig bei diesem Verfahren ist, daß die Anzahl der Reaktivgruppen in der Regel beschränkt ist durch den begrenzten Anteil der Reaktivmonomere im Polymer. Die Belegungsdichte der Rezeptoren auf der Oberfläche ist dadurch oftmals zu gering.

Ein weiterer Nachteil bei der Konzeption von Biosensoren ist die oft unspezifische Wechselwirkung von Proteinen mit der Festphasenoberfläche. Diese führt zur Adsorption mittels hydrophober oder ionischer Interaktionen, ist unerwünscht, führt zu nicht reproduzierbaren Ergebnissen und zur Verringerung der Meßgenauigkeit.

Die Erfindung betrifft einen optischen Festphasenbiosensor mit Biomolekülen als Rezeptoren zur spezifischen Erkennung von Analyten unter Ausnutzung des Förster-Energietransfers zwischen zwei Fluoreszenzfarbstoffen F1 und F2, bestehend aus
a) einem transparenten Träger,
b) einer daraufliegenden Multischicht, die abwechselnd aus Polyanionen und Polykationen besteht und als oberste Schicht ein biotinyliertes Polykation enthält, wobei der Grad der Biotinylierung 20-80 Mol-%, bevorzugt 30-70 Mol-%, besonders bevorzugt 40-60 Mol-%, bezogen auf die Zahl der Äquivalente kationischer Gruppen, beträgt,
c) einer Abdeckung der obersten biotinylierten kationischen Schicht durch Streptavidin, welches an diese biotinylierte Schicht gebunden wird,
d) weiteren biotinylierten Biomolekülen als Rezeptoren, bevorzugt Antikörpern, die mit einem Fluoreszenzfarbstoff F2 markierte Analyten binden können, wobei der Fluoreszenzfarbstoff F1 an die polyionischen Basisschichten, an das Streptavidin oder an die weiteren Antikörper bindenden Biomoleküle oder an die Antikörper gebunden sein kann.

In einer bevorzugten Ausführungsform sind an die unter c) beschriebene Schicht biotinylierte Rezeptoren gebunden, die ihrerseits Antikörper durch eine spezifische Erkennungsreaktion immobilisieren können.

Gegenstand der vorliegenden Erfindung ist demnach die Immobilisierung von Biomolekülen, insbesondere von Rezeptoren oder Antikörpern, an eine Festphase, wobei die Immobilisierung dauerhafter und gerichteter Natur ist und eine hohe Belegungsdichte der Oberfläche mit dem Rezeptor erreicht wird. Die Detektion der Bindung des Analyten erfolgt durch Förster-Energietransfer und ist durch eine molekular wohldefinierte gegenseitige Anordnung von Energie-Donor und -Akzeptor reproduzierbar und in ihrer Konzentrationsabhängigkeit regulär. Die Oberfläche wird gleichzeitig gegen unspezifische Adsorption von Proteinen passiviert. In der vorliegenden Erfindung wird demnach diesen Anforderungen nachgekommen und werden die oben angeführten Probleme gelöst durch die Immobilisierung organischer und biologischer Komponenten in molekular wohldefinierten Schichten unter Verwendung des natürlichen Systems Biotin/Streptavidin. Streptavidin ist ein Protein mit vier Bindungsstellen für Biotin (Vitamin H). Es kann deshalb als Matrix für die Ankopplung biotinylierter Biomoleküle dienen. Da die Bindungskonstante von Biotin mit Streptavidin ∼ 10¹⁵ M⁻¹ beträgt, ist die Bindung von Biotin an Streptavidin nahezu irreversibel.

Die Erfindung ist vor allem gekennzeichnet durch die Verwendung von Polykationen und Polyanionen zum Aufbau der Multischicht.

Realisiert wird diese Erfindung durch die Belegung eines transparenten festen Trägers, in der Regel Floatglas oder Quarzglas oder organische Polymere wie z.B. Polyester, Polycarbonat oder Polyethylenterephthalat oder andere transparente, nichtfluoreszierende Festkörper, mit einer Multischicht mittels der Self-Assembly-(SA-)Technik durch konsekutive Physisorption von anionischen und kationischen Polymeren. Dies Verfahren ist in EP 472 990 ausführlich beschrieben. In der vorliegenden Erfindung wird als letzte Schicht der Multischicht ein Polykation, welches an Aminogruppen biotinyliert ist, physisorbiert. Die Biotinylierung der Polykationen erfolgt mit Biotin-N-Hydroxysuccinimidester oder anderen reaktiven Estern zu 20-80 Mol-%, bevorzugt zu 30-70 Mol-%, besonders bevorzugt zu 40-60 Mol-%, bezogen auf die Zahl der Äquivalente kationischer Gruppen, gemäß dem in EP-A 0 472 990 beschriebenen Verfahren. Polykationen, die für die Erfindung geeignet sind, sind beispielsweise Polylysin, Polyallylamin, Polyvinylamin, Poly-(4-vinylpyridin), Polyacrylamid, Polymethacrylamid, Polyarginin, Polyasparagin, Polyglutamin, Polyethylenimin und Copolymere der zugrundeliegenden Monomere, bevorzugt Polylysin und Polyallylamin. Diese Polykationen, bei denen beispielsweise N-Atome als Ammoniumgruppen vorliegen, die 2 oder 3 H-Atome tragen, können als Gegenionen beispielsweise tragen: Halogenid, wie Chlorid oder Bromid, Sulfat, Hydrogensulfat, Nitrat, Nitrit, Carbonat, Hydrogencarbonat, Phosphat, Hydrogenphosphat, aliphatische Carbonsäureanionen, wie Formiat, Acetat, Trifluoracetat, Trichloracetat. Als biotinylierbare Polykationen kommen erfindungsgemäß in Frage: Polylysin, Polyarginin, Polyglutamin, Polyasparagin, Polyacrylamid, Polymethacrylamid, Polyallylamin und Copolymere der zugrundeliegenden Monomere, bevorzugt Polylysin und Polyallylamin. Polyanionen sind beispielsweise Polystyrolsulfonat (PSS), Polyacrylsäure, Polymethacrylsäure, Poly-(2-acrylamido-2-methyl-1-propansulfonsäure), Polyvinylsulfonsäure, Polyvinylsulfat, Dextransulfat, Cellulosesulfat und Copolymere der zugrundeliegenden Monomere, bevorzugt Polystyrolsulfonat. Gegenkationen in den Polyanionen sind beispielsweise H⁺, Na⁺, K⁺, NH₄⁺, bevorzugt Na⁺ oder K⁺. Der Anteil der biotinylierten Kationäquivalente kann über die Stöchometrie den gewünschten Erfordernissen angepaßt werden. Durch die Inkubation mit Streptavidin wird an die biotinylierte Polymerschicht Streptavidin gebunden. Die Oberfläche ist dann nahezu vollständig und, wie Fluoreszenzexperimente zeigen, lückenlos mit Streptavidin belegt. Ein solches System weist die oben geforderten Anforderungen und Vorteile für einen Biosensor auf. Zum einen wird die Biosensoroberfläche durch eine möglichst dichte Bedeckung mit dem Protein Streptavidin gegen unspezifische Wechselwirkungen abgeschirmt und zum anderen eine universelle Bindungsmatrix für eine Funktionalisierung der Festkörper-Grenzfläche zur Verwendung als Biosensor zur Verfügung gestellt. Durch die Markierung des Proteins Streptavidin mit einem Fluoreszenzfarbstoff F1 (z. B. mit Fluorescein-isothiocyanat), welche dem Fachmann hinlänglich bekannt ist, wird der Donorfarbstoff für den Förster-Energietransfer zur Verfügung gestellt.

Nach der Immobilisierung des Streptavidins an die biotinylierte Polymeroberfläche, dient nun das Streptavidin mit seinen noch nicht belegten Bindungsstellen als Matrix für die Anbindung von weiteren biotinylierten Biomolekülen als Rezeptoren, insbesondere für die Anbindung von Antikörpern. Mehrere bevorzugte Varianten zur Anbindung von Antikörpern sind möglich. So ist eine Ausführungsform der vorliegenden Erfindung die der Ankopplung von biotinylierten Protein A oder biotinyliertem Protein G an die Streptavidinmatrix. Die Biotinylierung von Protein A mittels N-Hydroxysuccinimid oder über andere reaktive Ester ist leicht möglich und dem Fachmann geläufig. Protein A ist ein Protein aus der Zellwand des Bakteriums Staphylococcus aureus. Es ist in der Lage, Immunglobuline vom Typ IgG spezifisch an deren F_{c}-Teil zu binden. Diese Methode hat zudem den Vorteil der regiospezifischen Immobilisierung von Antikörpern. Der F_{ab}-Teil der Antikörper bleibt frei. Eine Verminderung von immunologischer Aktivität durch Blockierung der Antigenbindungsstelle erfolgt nicht. Alternativ zur Bindung von F1 an das Streptavidin kann F1 auch an die soeben beschriebenen weiteren Biomoleküle gebunden werden; die Bindung an das Streptavidin ist jedoch bevorzugt.

Optische Festphasenbiosensoren dieser Art können beispielsweise in Form von Teststreifen eingesetzt werden.

Eine andere Ausführungsform des Immunosensors beinhaltet die Anbindung von biotinylierten Antikörpern an die Streptavidinmatrix. Hierbei sind wiederum zwei erfindungsmäßige Ausführungen denkbar. Zum einen kann die Biotinylierung der Antikörper mittels Biotin-N-Hydroxysuccinimid oder über andere reaktive Ester erfolgen. Eine solche dem Fachmann wohlbekannte Form der Biotinylierung von Antikörpern hat den Nachteil, daß die Biotinylierung nicht regiospezifisch erfolgt. Ein Teil der IgG-Moleküle wird auch an oder nahe der Antigenbindungsstelle biotinyliert, so daß eine sterische Blockierung derselben erfolgt und die immunologische Aktivität der Antikörper und damit die Sensitivität des Sensors abnimmt. In einer anderen erfindungsmäßigen Ausführungsform benutzt man Biotinderivate mit Hydrazidreaktivgruppen, die mit oxidierten Antikörpern reagieren. Bei der Oxidation der Antikörper werden die sich am F_{c}-Teil der IgGs befindlichen Kohlenhydrate in einer dem Fachmann bekannten Reaktion gespalten (Glykolspaltung) und Aldehyde erzeugt. Diese reagieren mit den Hydrazidgruppen der Biotinderivate unter Ausbildung von Hydrazonen. Die Biotingruppen sind dadurch regiospezifisch an den F_{c}-Teil der Antikörper angebunden. Die biotinylierten Antikörper werden an die Streptavidinmatrix gekoppelt und vervollständigen die Teststreifenoberfläche des Immunosensors, ohne daß eine Kopplung über biotinyliertes Protein A notwendig wird.

Der Teststreifen ist nun in der Lage, den mit einem geeigneten Akzeptorfarbstoff F2 (z. B. Rhodamin-isothiocyanat) versehenen Analyten durch Wechselwirkung des Rezeptors, insbesondere des Antikörpers, mit dem Analyten zu erkennen und zu quantifizieren. Der Nachweis des Analyten erfolgt durch einfaches Inkontaktbringen des beschichteten Trägers mit der Lösung, in der ein Molekül als Analyt vermutet wird (Probenlösung) und anschließender Fluoreszenzmessung. Es wird die Fluoreszenz des Donor- (F1) und des Akzeptorfarbstoffs (F2) gemessen. Befindet sich in der Testflüssigkeit (Probenlösung) ein mit dem Akzeptorfarbstoff F2 markierter Analyt, so wird nach dessen spezifischer Bindung an die immobilisierten Antikörper aufgrund des Förster-Energietransfers die Intensität der Akzeptorfluoreszenz zunehmen und die des Donors gegenüber dem ungebundenen Zustand abnehmen. Soll alternativ unmarkierter Analyt über eine Verdrängungsreaktion bestimmt werden, so wird der Teststreifen zuerst mit einem akzeptormarkierten Analog des betreffenden Analyten äquilibriert. In diesem Zustand überwiegt dann die Akzeptorfluoreszenz von F2 die Donorfluoreszenz von F1. Kommt anschließend unmarkierter Analyt aus der Testflüssigkeit mit dem äquilibrierten Teststreifen in Kontakt, so wird nach der Verdrängungsreaktion der Förster-Energietransfer unterbrochen sein, so daß eine Zunahme der Donorfluoreszenz von F1 und eine Abnahme der Akzeptorfluoreszenz von F2 die Bindung des unmarkierten Analyten signalisiert. In beiden Fällen hängt die Veränderung von Akzeptor- und Donorfluoreszenz in eindeutiger Weise mit der Konzentration des Analyten zusammen. Der Förster-Energietransfer läßt sich in üblichen Fluoreszenzspektrometern, aber auch in speziell ausgelegten Geräten für einen Energietransfer-Immunosensor, messen. Mittels geeigneter Kalibrationskurven läßt sich dann die Konzentration des Analyten in der Analysenflüssigkeit ermitteln. In einer weiteren Ausführungsform wird einer Analysenlösung, in der der zu detektierende Analyt vermutet wird, ein mit dem Fluoreszenzfarbstoff F2 markiertes, spezifisch bindendes Molekül in bekannter Menge zugesetzt, welches bei Vorhandensein das zu detektierenden Analyten mit diesem um die Bindung an der obersten Schicht des Biosensor kompetitiert. Die Konzentration des zu detektierenden Analyten wird sodann durch die Abhängigkeit der Fluoreszenzintensitäten von F2 bzw. von F1 oder dem Verhältnis der beiden Intensitäten gemessen.

Eine andere erfindungsmäßige Ausführungsform des Immunosensors beinhaltet die Anbindung des Donorfarbstoffes F1 an den Rezeptor, bevorzugt den Antikörper, vorzugsweise über Hydrazidreaktivgruppen an oxidierte Antikörper in der oben beschriebenen Form. Bei dieser Ausführungsform wird die Effizienz des Förster-Energietransfers durch eine Verringerung des durchschnittlichen Abstandes zwischen Donor(F1)- und Akzeptor(F2)-Farbstoff gesteigert, was zu einer Erhöhung der Sensitivität des Testverfahrens genutzt werden kann.

Die Erfindung wird durch die folgenden Abbildungen näher erläutert:

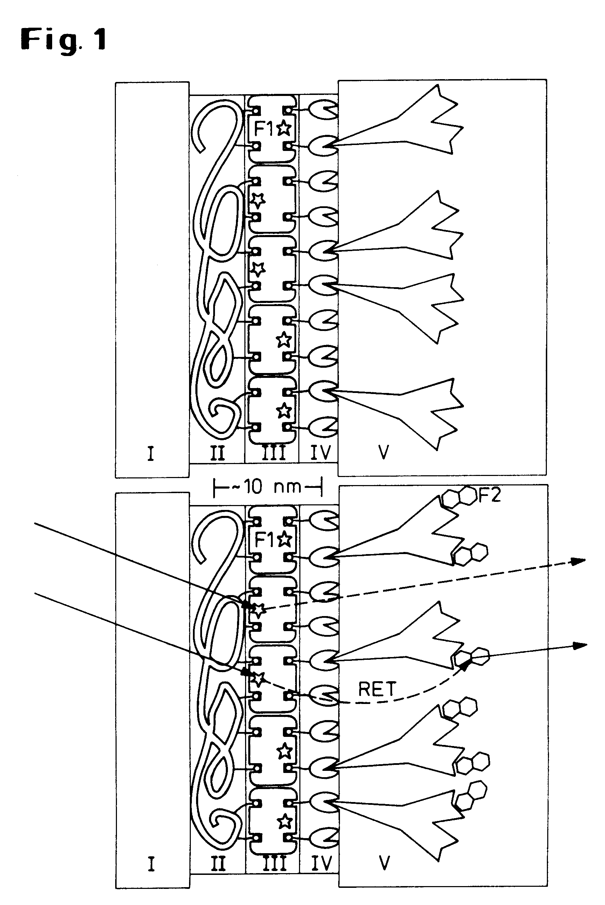

Figur 1 zeigt schematisch, und in etwa maßstabsgerecht, den Multischichtaufbau der Teststreifen für den Förster-Energietransfer-Immunosensor (oben), sowie das Detektionsprinzip (unten).
- Figur 1:: Schematischer Aufbau und Funktion des Immonunosensors. Die molekularen Komponenten sind in etwa maßstäblich gezeichnet. Der Träger I ist viel dicker als dargestellt.
- Oben:: Teststreifen, bestehend aus transparentem Träger I (z.B. Glas), einer Polyelektrolyt-Multischicht II (zur Vereinfachung der Abbildung ist nur die letzte biotinylierte Schicht gezeigt), einer Schicht mit F1 fluoreszenzmarkierten Streptavidins III, einer Schicht biotinyliertem Protein A IV und dem darauf immobilisierten Antikörper V.
- Unten:: Nach Benetzung mit Testflüssigkeit hängt die Wellenlänge der beobachteten Fluoreszenz nach Anregung des an Streptavidin gekoppelten Fluoreszenzfarbstoffs F1 davon ab, ob direkte Emission vorliegt (punktierter Pfeil) oder ob die Anregungsenergie nach Energietransfer (RET = gestrichelter Pfeil) auf den markierten Analyten rotverschoben emittiert wird (durchgezogener Pfeil). Das Verhältnis von rotverschobener zu direkter Emissionsintensität ist in eindeutiger Weise abhängig von der Anzahl gebundener Analytmoleküle pro Flächeneinheit.

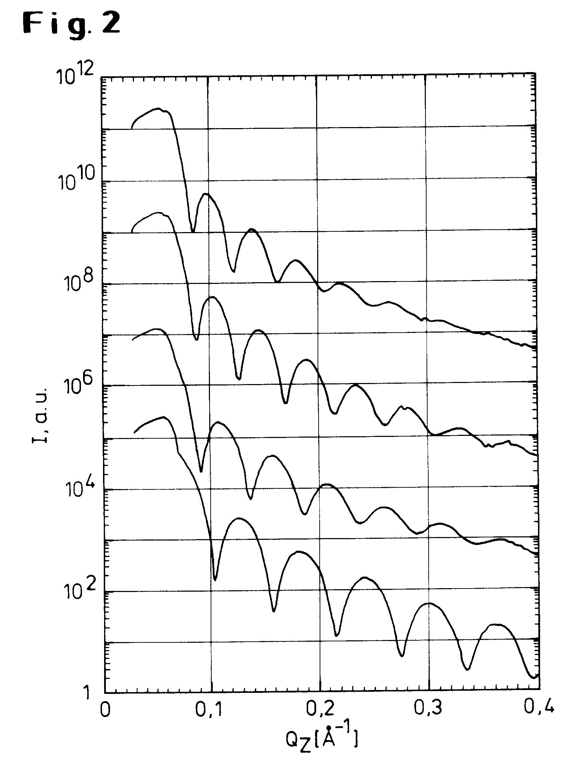

Figur 2 zeigt Röntgen-Reflektogramme der Schichtstruktur in verschiedenen Stadien der Präparation.
- Figur 2:: Röntgen-Reflektogramme der Schichtstruktur (Trägermaterial : Silizium) in verschiedenen Stadien der Präparation. Die verschiedenen Datensätze wurden um jeweils einen Faktor 100 gegeneinander verschoben. Die Kurven zeigen von unten nach oben zunächst die Polyelektrolyt-Multischicht, dann dieselbe, belegt mit Streptavidin, anschließend noch belegt mit biotinyliertem Protein A und zum Schluß versehen mit Antikörper IgG. Auf der Abszisse ist der Impulstransfer Q_{z} in Å⁻¹ und auf der Ordinate die Röntgen-Intensität I aufgetragen.

Die Messung zeigt für das vorliegende Beispiel, daß die Oberfläche mit einer in den verschiedenen Präparationsschritten regulär gewachsenen Grenzflächenschicht organischen Materials belegt ist und in jedem Schritt die Präparation molekular glatt bleibt. Dies bedeutet insbesondere, daß die Oberflächenfunktionalisierung nicht zur Belegung der Festphasengrenzfläche mit lateral inhomogenen Strukturen führt, d.h. eine Tröpfchenbildung findet selbst auf der Längenskala von Nanometern nicht statt.

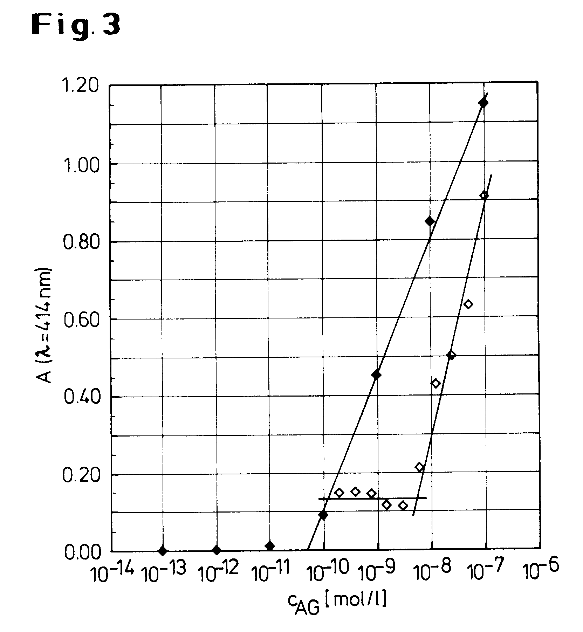

Figur 3 zeigt die Ergebnisse von ELISA-Messungen an Sensoroberflächen, die analog zu dem in Figur 1 angedeuteten und in Figur 2 mittels Röntgenreflektivitätsmessungen protokolliertem Verfahren hergestellt wurden.
- Figur 3:: Titration verschiedener Sensoroberflächen mit Antigen. Die spezifische Bindung wurde mittels ELISA nachgewiesen. Einem Sensor, der nach der hier beschriebenen Technik präpariert wurde (schwarze Karos), ist ein Präparat gegenübergestellt, bei dem IgG elektrostatisch an eine PSS-Schicht adsorbiert wurde (weiße Karos). Auf der Abszisse ist die Antigenkonzentration c_{AG} in mol/l und auf der Ordinate die optische Dichte A bei λ = 414 nm angegeben.

Figur 4 zeigt die Ergebnisse einer Biosensor-Messung an einer Probe, die wie die in Figur 3 beschriebene Probe hergestellt wurde.

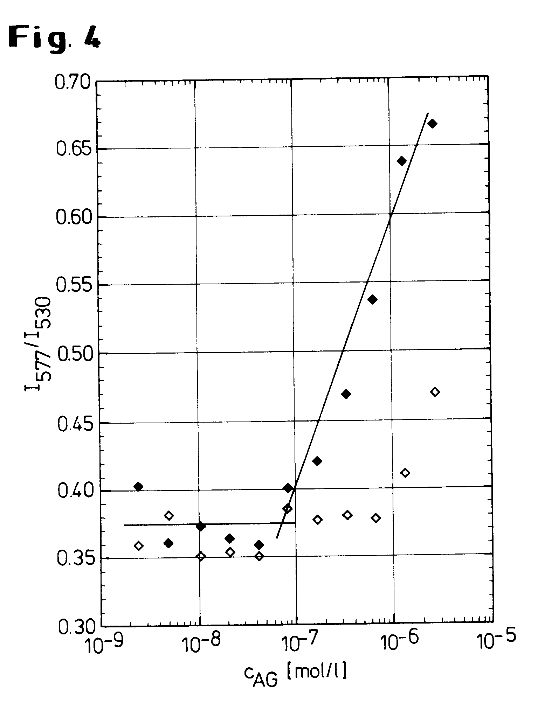

- Figur 4:: Titration einer Sensoroberfläche mit Antigen (schwarze Karos). Die spezifische Bindung wurde mittels Energietransfer nachgewiesen. Das Antigen lag in ca. 2facher Verdünnung im Kulturüberstand vor. Kontrolle (weiße Karos): Antigenadsorption an Teststreifen, der nur bis zur Streptavidinschicht aufgebaut war (ohne Protein A und Rezeptorschicht). Die Abszisse zeigt die Konzentration c_{AG} im Kulturüberstand in mol/l. Die Ordinate zeigt das Intensitätsverhältnis der Fluoreszenzen der Fluoreszenzfarbstoffe F1 und F2 bei 577 und 530 nm.

### Beispiele:

### 1. Verwendete Chemikalien

- **Polymere**:: Polystyrolsulfonat, Natriumsalz (PSS), MW = 70.000, Aldrich
Polyallylamin-hydrochlorid (PAH), MW = 50.000―60.000, Aldrich
Polyethylenimin (PEI), MW =50.000, 50%ige Lösung in H₂O, Aldrich
Poly-L-Lysin-hydrobromid (PL), MW < 50.000, Bachem-Biochemica

Das PSS wurde in wässriger Lösung in einem Dialyseschlauch VISKING27/32 der Firma Roth zwei Tage gegen Reinstwasser dialysiert und anschließend gefriergetrocknet.
- **Proteine**:: Streptavidin, Boehringer-Mannheim
Protein A, Pharmacia
Kaninchen IgG, polyclonal, Spezifität: Anti Maus IgG,
Immunol. Institut Univ. Mainz
Bovine Serum Albumin (BSA), Sigma
- **Antigene:**: Maus-IgG, monoclonal, Kulturüberstand, Immunol. Institut Univ. Mainz
Horseradish-Peroxidase-(HPO-) gekoppeltes Maus-IgG, affinitätsgereinigt, Jackson Immuno-Research Laboratories, Dianova, U.S.A.
- **Fluorophore:**: Rhodamin-B-Isothiocyanat (RITC), Sigma
Fluorescein-Isothiocyanat Isomer I (FITC), Sigma

Die Markierung des Streptavidin betrug durchschnittlich 1,4 FITC pro Protein-Molekül und die des Maus-IgG durchschnittlich 3 RITC pro Protein-Molekül.
- **Biotinaktivester**:: Biotinamidocaproyl-N-Hydroxysuccinimidester, Sigma

Zur Biotinylierung von Protein A wurden der Aktivester und das Protein in einem molekularen Verhältnis von 12:1 eingewogen.
- **Detergens**:: Polyoxyethylensorbitan-Monolaurat (TWEEN-20), Sigma
- **PBS-Puffer:**: Natriumdihydrogenphosphat, Monohydrat, p.a., Merck
Dinatriumhydrogenphosphat, p.a., Merck
Natriumchlorid, p.a., Merck
- **Citrat-Puffer:**: Dinatriumhydrogenphosphat, p.a., Merck
Zitronensäuremonohydrat, Sigma
Kaliumchlorid, p.a., Merck
Magnesiumchlorid, Hexahydrat, p.a., Merck
- **ABTS:**: 2,2'-Azino-Bis(3-Ethyl-Benzthiazolin)-Sulfonsäure, Sigma
- **Glassubstrat:**: (38 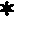 12) mm² große und (1―1,2) mm dicke Objektträger, Fa. Gebr. Rettberg GmbH.

### 2. Trägerreinigung

Die Trägerreinigung erfolgte entsprechend einer Standardprozedur (W. Kern, D. A. Puotinen, RCA Review, 31 (1970), 187)

### 3. Trägerpräparation

Alle Lösungen wurden mit destilliertem Wasser angesetzt. Die wasserbenetzten Träger wurden für 30 Minuten in PEI-Lösung (verdünnt auf 2,2 mg/ml) eingestellt und anschließend dreimal für jeweils ca. 30 Sekunden in 10 ml Wasser gewaschen und anschließend in einem schwachen Stickstoffstrom trocken geblasen. Anschließend wurde die Probe für ca. 20 Minuten in eine PSS-Lösung (20 mg PSS in 10 ml einer 2 M NaCl-Lösung) gestellt und wie oben beschrieben gewaschen und getrocknet. Der Träger wurde für weitere 20 Minuten in eine PAH-Lösung (20 mg PAH in 10 ml 2 M NaCl-Lösung) eingestellt und wieder gewaschen und getrocknet. Wie oben beschrieben wurden eine weitere PSS-, eine PAH- und wiederum eine PSS-Schicht an den Träger adsorbiert. Zur Funktionalisierung mit Biotin wurde der Träger in eine Lösung von biotinyliertem Polylysin-hydrobromid (PLB) (5 mg/10 ml in 0,4 M NaCl) für 20 Minuten eingestellt und danach wieder gewaschen und getrocknet. Die beschichteten Träger wurden bis zur Verwendung bei einer Temperatur von 4° C aufbewahrt.

### 4. Aufbau der Protein-Heteromultischichten:

PLB-beschichtete Träger wurden für ca. 30 Minuten in eine FITC-Streptavidinlösung (10⁻⁷ mol/l FITC-Streptavidin in 10 mM PBS-Puffer, pH=7,2, 150 mM NaCl) gestellt, dreimal mit 10 ml Wasser gewaschen und anschließend für weitere 40 Minuten in eine Lösung von biotinyliertem Protein A (5 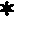 10⁻⁷ mol/l, PBS-Puffer wie oben) eingestellt und dreimal mit dem reinen PBS-Puffer gewaschen. Auf Trocknungsschritte zwischen den einzelnen Proteinbeschichtungen wurde verzichtet. Der mit Protein A beschichtete Träger wurde für 40 Minuten in eine Kaninchen IgG-Lösung (Spezifität: Anti-Maus IgG, 5 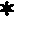 10⁻⁷ mol/l, PBS-Puffer wie oben) gestellt und dreimal mit dem reinen Puffer gewaschen. Bis zum Anbinden des Antigens wurde der Träger in PBS-Puffer aufbewahrt.

Figur 2 zeigt Röntgen-Reflektogramme, die bei der Präparation eines Teststreifens (Trägermaterial Silizium) zwischen den verschiedenen Adsorptionsschritten gemessen wurden (trockene Probe). Diese Ergebnisse belegen, daß sich beim Adsorptionsprozeß eine kohärente, auf molekularer Längenskala gleichmäßige Schichtstruktur ausbildet, daß die Schichtdickeninkremente jeweils den molekularen Dimensionen der Absorbentien entsprechen und daß die Oberfläche nach jedem Adsorptionsschritt molekular glatt bleibt. Eine Ausnahme bildet die terminale Antikörperschicht, die wegen der elongierten Molekülform des Antikörpers und der regioselektiven Bindung durch Protein A einen hohen Anteil von wässrigem Puffer enthält. Diese Schicht kollabiert beim Trocknungsprozeß während der Messung und erscheint danach wesentlich dünner als dies die molekularen Dimensionen erwarten lassen. Die experimentellen Daten aus Figur 2 sind in Tabelle 1 quantitativ ausgewertet.

**Tabelle 1**

| Molekulare Dimensionen der aktiven Grenzflächenschicht des Immunosensors | | |
|---|---|---|
| Beschichtungsschritte | Schichtdickeninkrement (Å) | Oberflächenrauhigkeit org. Grenzfläche/Luft (Å) |
| 6 molekulare Polyelektrolytschichten (inkl. PLB) | 203 | 6 |
| Streptavidinschicht auf Polyelektrolytfilm/PLB | 56 | 11 |
| Biotinyliertes Protein A auf Streptavidinschicht | 7 | 11 |
| Kaninchen IgG (Anti-Maus) auf Protein A | 13 | 17 |

### 5. Inkubation mit Antigen, einschließlich Kontrollen

### a. ELISA-Messungen

Proben wurden, wie oben beschrieben, auf Silizium präpariert. In PBS-Puffer mit 0.1% TWEEN-20 wurden 10 mg/ml BSA gelöst. Diese Proteinlösung wurde für ELISA-Messungen zur Herstellung einer Verdünnungsreihe des HPO-markierten Antigens (Maus-IgG-HPO) verwendet, dessen Konzentration zwischen 10⁻¹³ und 10⁻⁷ mol/l betrug. Nach Inkubation mit dem Antigen wurden die Proben in Citrat-Puffer mit 3 g/l ABTS und 0.0075 % H₂O₂ entwickelt und vermessen. Figur 3 zeigt ein repräsentatives Ergebnis für ein Präparat, das nach der hier vorgestellten Technologie hergestellt wurde. Dem gegenübergestellt ist die Titration eines Präparats, bei dem IgG lediglich elektrostatisch an eine Silizium-Grenzfläche adsorbiert wurde, die mit einer molekular dünnen Schicht von PSS bedeckt war. Deutlich wird in der Abbildung die höhere Empfindlichkeit, bessere Linearität und geringere unspezifische Adsorption sichtbar, die das nach der neuen Technik hergestellte Präparat auszeichnen.

### b. Fluoreszenz-Messungen

Proben wurden, wie oben beschrieben, auf Floatglas präpariert. In PBS-Puffer wurden 10 mg/ml BSA gelöst. Diese Proteinlösung wurde zur Herstellung einer Verdünnungsreihe des RITC-markierten Antigens verwendet. In dieser Verdünnungsreihe betrug die Konzentration des Antigens zwischen 2,5 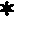 10⁻⁹ und 5 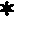 10⁻⁶ mol/l. Die Träger wurden ca. 40 Minuten in die Lösung einer bestimmten Antigenkonzentration eingestellt und anschließend 5 mal ca. 1 Minute in einem mit 0.1% TWEEN-20 versetzten PBS-Puffer (Puffer-Zusammensetzung wie oben) gewaschen. Die Träger wurden im Stickstoffstrom trockengeblasen und bis zum Vermessen in Dunkelheit bei 4°C aufbewahrt.

Zur Quantifizierung der unspezifischen Wechselwirkung des Antigens mit dem Substrat wurden Träger in der gleichen Sequenz bis einschließlich zur Streptavidinschicht (aber ohne Protein A- und IgG-Schichten) beschichtet und für ca. 40 Minuten in jeweils eine antigenhaltige Lösung der Verdünnungsreihe gestellt und, wie oben beschrieben, gewaschen und getrocknet.

Die Proben- und Referenzträger wurden sowohl in einem herkömmlichen Fluoreszenzspektrometer als auch einem speziell ausgelegten Gerät zur Messung des Fluoreszenzenergietransfer im trockenen Zustand vermessen. Figur 4 zeigt repräsentative Ergebnisse.

## Patentansprüche

1. Optischer Festphasenbiosensor mit Biomolekülen als Rezeptoren zur spezifischen Erkennung von Analyten unter Ausnutzung des Förster-Energietransfers zwischen zwei Fluoreszenzfarbstoffen F1 und F2, bestehend aus
a) einem transparenten Träger,
b) einer daraufliegenden Multischicht, die abwechselnd aus Polyanionen und Polykationen besteht und als oberste Schicht ein biotinyliertes Polykation enthält, wobei der Grad der Biotinylierung 20-80 Mol-%, bevorzugt 30-70 Mol-%, besonders bevorzugt 40-60 Mol-%, bezogen auf die Zahl der Äquivalente kationischer Gruppen, beträgt,
c) einer Abdeckung der obersten biotinylierten kationischen Schicht durch Streptavidin, welches an diese biotinylierte Schicht gebunden wird,
d) weiteren biotinylierten Biomolekülen als Rezeptoren, bevorzugt Antikörpern, die mit einem Fluoreszenzfarbstoff F2 markierte Analyten binden können, wobei der Fluoreszenzfarbstoff F1 an die polyionischen Basisschichten, an das Streptavidin oder an die weiteren Antikörper bindenden Biomoleküle oder an die Antikörper gebunden sein kann.

2. Optischer Biosensor gemäß Anspruch 1, in dem der Donor-Farbstoff F1 an Streptavidin gebunden ist.

3. Optischer Biosensor gemäß Anspruch 1 oder 2, bei dem Rezeptoren, bevorzugt die Antikörper, über biotinylierte Biomoleküle, die selbst keine Antikörper sind, insbesondere über Protein A oder Protein G, an die Streptavidin-Schicht gebunden sind.

4. Optischer Biosensor gemäß Anspruch 1 oder 2, bei dem Rezeptoren, bevorzugt Antikörper, die biotinyliert sind, an die Streptavidin-Schicht gebunden sind.

5. Optischer Biosensor gemäß Anspruch 1-4, in dem das Analytmolekül ein anderes mit dem Fluoreszenzfarbstoff F2 markiertes, an die oberste Schicht gebundenes Molekül, das nicht der Analyt ist, von der obersten Schicht verdrängt und die Konzentration des schließlich gebundenen Analytmoleküls in Abhängigkeit von der Abnahme der Fluoreszenzintensität von F2 bzw. der Zunahme der Fluoreszenzintensität von F1 oder der Änderung des Verhältnisses von beiden Intensitäten gemessen wird.

6. Optischer Biosensor gemäß Anspruch 1-4, in dem das Analytmolekül mit einem mit dem Fluoreszenzfarbstoff F2 markierten, spezifisch bindendes Molekül, das nicht der Analyt ist und welches der Analysenlösung in bekannter Menge zugesetzt wird, um die Bindung an die oberste Schicht des Biosensors kompetitiert, und die Konzentration des Analyten in Abhängigkeit von der Fluoreszenzintensität von F2 bzw. von F1 oder dem Verhältnis der beiden Intensitäten gemessen wird.

7. Optischer Biosensor gemäß Anspruch 1-6, in dem als Trägermaterial Floatglas, Quarzglas, Polyester, Polyethylenterephthalat oder Polycarbonat oder andere transparente, nichtfluoreszierende Festkörper eingesetzt werden.
